# EUROPEAN PATENT APPLICATION

(11) **EP 4 215 607 A1**
(43) Date of publication of application: **26.07.2023**
(21) Application number: 21869773.8
(22) Date of filing: 17.09.2021
(51) Int. Cl.: C12N 9/16, A61K 38/43, A61P 35/00, A61K 38/00

(54) **PTPSIGMA-FC FUSION PROTEIN AND PHARMACEUTICAL COMPOSITION COMPRISING SAME**

(30) Priority: 18.09.2020 KR 20200120887; 03.09.2021 KR 20210117865
(71) Applicant: IUCF-HYU (Industry-University Cooperation Foundation Hanyang University), Seoul 04763 (KR)
(72) Inventor: RYU, Seong Eon, Seoul 07225 (KR); JANG, Hye Hyeon, Seoul 04762 (KR); KIM, Myeong Bin, Seoul 04808 (KR); PARK, Sung Ho, Daejeon 34140 (KR)
(74) Representative: dompatent von Kreisler Selting Werner - Partnerschaft von Patent- und Rechtsanwälten mbB
(86) International application number: PCT/KR2021/012763
(87) International publication number: WO 2022/060151

(57) **Abstract**

The present invention relates to a tyrosine phosphatase sigma (PTPsigma)-Fc fusion protein comprising a PTPsigma-derived protein and an immunoglobulin (Ig)-derived Fc domain, which can inhibit the signal transduction of PTPsigma by blocking the interaction of the extracellular domain of PTPsigma with a ligand. The PTPsigma-Fc fusion protein of the present invention inhibits PTPsigma-mediated signaling and as such, can promote the growth of blood stem cells, and can be used for preventing or treating PTPsigma-mediated diseases.

Controlling the signal transduction of PTPsigma by utilizing interaction between proteins, the PTPsigma-Fc fusion protein according to the present invention can overcome the problems of conventional inhibitors targeting the active site of PTP. In addition, the solubility of the fusion protein in an aqueous solution can be improved by introducing a mutation thereinto, which makes it possible to apply a high dose to the treatment of the human body and animals.

## Description

### [Technical Field]

The present invention relates to a PTPsigma-Fc fusion protein and a pharmaceutical composition comprising the same, and more specifically, to a PTPsigma-Fc fusion protein which interferes with the interaction between an ectodomain of PTPsigma and ligands, thereby capable of being used to promote the growth of blood stem cells and prevent or treat a PTPsigma-mediated disease, and a pharmaceutical composition comprising the same.

### [Background Art]

Proteins play a very important role in signaling for intracellular function control. Signaling by proteins is achieved by controlling the amount or activity of proteins, and various modifications such as phosphorylation, glycosylation, methylation and acetylation occur to induce activation or inactivation of proteins.

Among them, protein phosphorylation plays a key role in controlling various cellular phenomena such as a cell growth, a differentiation, an immune response and a brain function. Inadequate control of protein phosphorylation causes many diseases such as a cancer, diabetes, an immune disease and a nervous system disease.

Thus, protein kinases and protein phosphatases that control protein phosphorylation and dephosphorylation are important target proteins for the development of therapeutic agents for diseases. While inhibitors of protein kinases have already been clinically proven to be effective as therapeutic agents for diseases, research on therapeutic agents for diseases targeting protein phosphatases has recently been actively conducted, and no successful therapeutic agents have been reported in clinical practice yet.

Protein tyrosine phosphatase (PTP) is an enzyme that hydrolyzes a phosphate group on a phosphorylated tyrosine residue. The phosphorylation-dephosphorylation process of tyrosine is used as a very important means in the intracellular signaling system, and in particular, plays a key role in a cell response to external stimuli, and a growth, differentiation and death of a cell. Thus, PTP occupies an important position as a therapeutic target protein for diseases such as a cancer, a vascular disease, an immune disease and a neurological disease, accompanied by changes in the cell state as described above. As an example of a PTP inhibitor, Korean Patent No. 10-1194968 describes a technique for preventing or treating PTP1B-mediated diseases such as diabetes by inhibiting the activity of PTP1B using 1,1'-(1,2-ethyndiyl)bis-benzene derivatives, and Korean Patent No. 10-1826690 describes a therapeutic agent for neuroinflammatory diseases using PTP inhibitors targeting PTP1B, TC-PTP, SHP2, LYP, and RPTPβ.

The development of these conventional PTP inhibitors mainly targeted the active site of the PTP enzyme. However, the PTP active site pocket has a relatively shallow depth, a narrow part capable of strong interaction with the inhibitor compound, and similar enzymatic reaction domains between PTPs, and thus, it is difficult for the inhibitor compound to have selectivity. In addition, the PTP active site pocket is positively charged, and thus, compounds that bind to it become negatively charged to mimic the phosphate group, which is the substrate of the PTP enzymes, and such charged compounds have difficulty passing through cell membranes.

That is, conventional PTP inhibitors targeting the active site of PTP have limitations in that they are difficult to optimize in terms of inhibitory strength of enzyme function, selectivity, permeability of a cell membrane, and the like. Accordingly, there is a demand for the development of a therapeutic agent capable of overcoming these limitations.

On the other hand, signaling of a receptor-type PTP such as PTPsigma can be achieved by the receptor-ligand interaction between an extracellular domain (ectodomain) and its ligand in addition to the active site. The receptor-ligand interaction of PTPsigma is known to be involved in signaling such as inhibition of nerve cell regeneration, and Parkinson's disease and the like are known as a PTPsigma-mediated disease.

Under the circumstances, the present inventors studied to develop a PTPsigma inhibitor capable of overcoming the limitations of inhibitory strength of enzyme function, selectivity, permeability of a cell membrane, and the like of conventional PTP inhibitor compounds. As a result, there has been developed a PTPsigma-Fc fusion protein capable of overcoming the limitations of conventional PTP inhibitor compounds while inhibiting PTPsigma signaling by interfering with the receptor-ligand interaction that occurs in the extracellular domain of PTPsigma using the interaction between proteins.

### [Disclosure]

### [Technical Problem]

It is an object of the present invention to provide a PTPsigma-Fc fusion protein capable of inhibiting PTPsigma signaling by blocking the interaction between an extracellular domain of PTPsigma and its ligand.

It is another object of the present invention to provide a composition for promoting the growth of blood stem cells, comprising the PTPsigma-Fc fusion protein.

It is another object of the present invention to provide a pharmaceutical composition for preventing or treating a PTPsigma-mediated disease, comprising the PTPsigma-Fc fusion protein.

### [Technical Solution]

In order to achieve the above objects, the present invention provides a PTPsigma-Fc fusion protein comprising a PTPsigma (protein tyrosine phosphatase sigma)-derived protein linked to one or more of two chains constituting an immunoglobulin (Ig)-derived Fc domain.

In the present invention, the PTPsigma-derived protein may be a protein derived from an extracellular domain (ectodomain) of PTPsigma.

In the present invention, the PTPsigma-derived protein may be a protein derived from the first to second Ig domains (Ig1-2) or the first to third Ig domains (Ig1-3) of the extracellular domain of PTPsigma.

In the present invention, the PTPsigma-derived protein may comprise amino acid residues 30-231 of a PTPsigma protein.

In the present invention, one or more residues of isoleucine (I), valine (V), leucine (L) and tyrosine (Y) in the PTPsigma-derived protein may be substituted with alanine (A), serine (S), threonine (T), asparagine (N), glutamine (Q), aspartic acid (D), glutamic acid (E), lysine (K), arginine (R), or histidine (H).

In the present invention, one or more residues of I36, V104, V108, L125, L143, L145, L155, L187, Y224 and V227 based on the amino acid sequence number of a PTPsigma protein in the PTPsigma-derived protein may be substituted with A, S, T, N, Q, D, E, K, R, or H.

In the present invention, the PTPsigma-derived protein may be in the form of a homodimer or a heterodimer.

In the present invention, the chains constituting the Fc domain may comprise amino acid residues 221-447 (based on Kabat EU numbering) of an IgG1 heavy chain.

In the present invention, one or more amino acids in one chain of the Fc domain may be substituted with an amino acid selected from the group consisting of tryptophan (W), arginine (R), phenylalanine (F), and tyrosine (Y); and one or more amino acids in the other chain may be substituted with an amino acid selected from the group consisting of alanine (A), serine (S), threonine (T), and valine (V).

In the present invention, one chain of the Fc domain may comprise T366W substitution (based on Kabat EU numbering); and the other chain may comprise T366S, L368A and Y407V substitutions (based on Kabat EU numbering).

The present invention also provides a composition for promoting the growth of blood stem cells, comprising the PTPsigma-Fc fusion protein.

In the present invention, the composition for promoting the growth of blood stem cells may be used for regenerating blood stem cells damaged by irradiation or administration of anticancer drugs.

The present invention also provides a method of promoting the growth of blood stem cells, comprising administering a pharmaceutical composition comprising the PTPsigma-Fc fusion protein.

The present invention also provides a pharmaceutical composition comprising the PTPsigma-Fc fusion protein.

In the present invention, the pharmaceutical composition may be used for preventing or treating a PTPsigma-mediated disease.

In the present invention, the PTPsigma-mediated disease may be a nerve injury disease selected from the group consisting of traumatic brain injury, Alzheimer's dementia, and Parkinson's disease.

In the present invention, the PTPsigma-mediated disease may be selected from the group consisting of multiple sclerosis and rheumatoid arthritis.

In the present invention, the PTPsigma-mediated disease may be a synaptic plasticity-related disease selected from the group consisting of mania, depression, manic depression, and amnestic disorder.

The present invention also provides a method of preventing or treating a PTPsigma-mediated disease, comprising administering a pharmaceutical composition comprising the PTPsigma-Fc fusion protein.

### [Advantageous Effects]

In the present invention, the reaction between an extracellular domain of PTPsigma and its ligand may be blocked by using a fusion protein comprising a PTPsigma-derived protein bound to a Fc region of immunoglobulin. According to the present invention, the regulator using the interaction between proteins is used, and thus, it is possible to overcome the limitations of inhibitory strength of enzyme function, selectivity, permeability of a cell membrane, and the like of conventional inhibitors targeting the active site of PTP. In addition, it may be treated at a high dose in the treatment of humans and animals by introducing mutations into the fusion protein to improve its solubility in aqueous solution.

The PTPsigma-Fc fusion protein of the present invention may promote the growth of blood stem cells by inhibiting PTPsigma-mediated signaling and may be used to prevent or treat a PTPsigma-mediated disease.

### [Description of Drawings]

FIG. 1 shows a schematic design of (a) homodimer and (b) heterodimer of the PTPsigma-Fc fusion protein of the present invention.
FIG. 2 shows an amino acid sequence map of the PTPsigma-Fc fusion protein according to an embodiment of the present invention.
FIG. 3 shows an amino acid sequence map of a solubility-enhancing mutant of the PTPsigma-Fc fusion protein according to an embodiment of the present invention.
FIG. 4 shows an amino acid sequence encoded by an expression vector for preparing the PTPsigma-Fc homodimer according to an embodiment of the present invention.
FIG. 5 shows an amino acid sequence encoded by an expression vector for preparing the PTPsigma-Fc heterodimer according to an embodiment of the present invention.
FIG. 6 shows the results of non-reducing SDS-PAGE after preparation of the PTPsigma-Fc fusion protein according to an embodiment of the present invention.
FIG. 7 is a graph showing the effect of regenerating hematopoietic stem cells by the PTPsigma-Fc fusion protein according to an embodiment of the present invention.
FIG. 8 shows the positions of residues to be mutated in the tertiary structure of PTPsigma Ig1-2.
FIG. 9 shows a structure in which the complex structure of Ig1-2 of PTP-lar and sucrose octasulfate and the tertiary structure of PTPsigma Ig1-2 are overlapped.
FIGS. 10 to 27 show amino acid sequences encoded by PTPsigma mutant-Fc expression vectors according to embodiments of the present invention.
FIG. 28 is a graph showing the yield and solubility of the PTPsigma-Fc fusion protein according to an embodiment of the present invention.
FIG. 29 is a graph showing the effect of regenerating hematopoietic stem cells by the PTPsigma-Fc fusion protein according to an embodiment of the present invention.

### [Best Mode]

Unless defined otherwise, all technical and scientific terms used in the present specification have the same meaning as commonly understood by those of ordinary skill in the technical field to which the present invention pertains. In general, the nomenclature used in the present specification is those well known and commonly used in the art.

The present invention relates to a PTPsigma-Fc fusion protein capable of inhibiting PTPsigma signaling by blocking the receptor-ligand interaction between an extracellular domain of PTPsigma and its ligand.

PTPsigma (protein tyrosine phosphatase sigma) is a transmembrane receptor type PTP, and is composed of three sites: an extracellular domain (ectodomain) site, a transmembrane site and a cytoplasmic site. Among these, the extracellular domain is composed of three Ig-like domains followed by several fibronectin domains, starting from the N-terminus. The extracellular domain site undergoes receptor-ligand interaction, and this interaction is transferred to the cytoplasmic site to control the enzyme activity of the PTP active domain.

On the assumption that the Ig-like domain in the extracellular domains of PTPsigma would be involved in the receptor-ligand interaction, the present inventors constructed a PTPsigma-Fc fusion protein by fusing the Ig-like domain to the Fc region of immunoglobulin, and tested the PTPsigma inhibitory ability of the fusion protein. As a result, it was confirmed that the radiation-treated blood stem cells were effectively regenerated when the blood stem cells was treated with the fusion protein. Accordingly, it was found that the PTPsigma-Fc fusion protein of the present invention may promote the regeneration of blood stem cells of PTPsigma.

Accordingly, the PTPsigma-Fc fusion protein of the present invention may be used in a composition for promoting the growth of blood stem cells, and in addition, may be used as a therapeutic agent for PTPsigma-mediated diseases such as a nerve injury disease, an autoimmune disease and a synaptic plasticity-related disease, like conventional PTPsigma inhibitors.

In addition, as a result of selecting amino acid residues that do not change the structure among the hydrophobic amino acids exposed on the surface of the Ig-like domain of PTPsigma and substituting them with hydrophilic amino acids in order to improve the solubility of the fusion protein, it was confirmed that the effect of regenerating blood stem cells was maintained while the solubility of the fusion protein increased.

The PTPsigma-Fc fusion protein of the present invention comprises a PTPsigma-derived protein and an Fc domain.

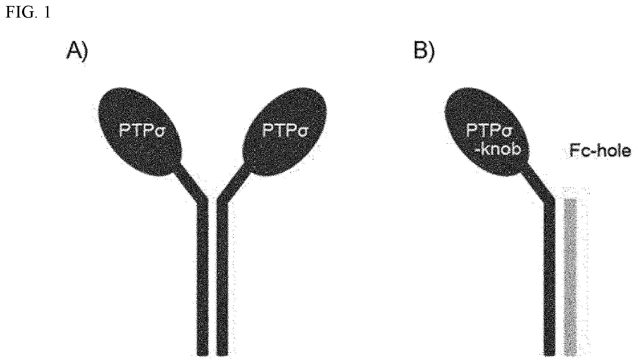

FIG. 1 shows a schematic design of the PTPsigma-Fc fusion protein according to the present invention. FIG. 1A shows a homodimer structure in which PTPsigma-derived proteins are linked to both chains of the Fc domain, and FIG. 1B shows a heterodimer structure in which the PTPsigma-derived protein is linked to only one chain of the Fc domain.

In the present invention, the parent of the PTPsigma-derived protein is interpreted as meaning including not only the standard amino acid sequence of the PTPsigma protein but also its isoform. The standard sequence of the PTPsigma protein refers to isoform 1 (Q13332-1) based on Unitprot, and other isoforms include isoform PTPS-MEA (Q13332-2), isoform PTPS-MEB (Q13332-3), isoform PTPS-MEC (Q13332-4), isoform PTPS-F4-7 (Q13332-5), isoform 2 (Q13332-6) and isoform 3 (Q13332-7), and a preferred parent sequence may be the sequence of PTPsigma isoform 3.

In the present invention, the PTPsigma-derived protein may be a protein derived from an extracellular domain (ectodomain) of PTPsigma. In this case, the protein derived from the extracellular domain of PTPsigma refers to a protein containing all or part of the sequence of the extracellular domain of PTPsigma.

In particular, the protein derived from the extracellular domain of PTPsigma in the present invention is preferably a protein derived from the Ig-like domain of the extracellular domain, among which is more preferably a protein derived from the first to second Ig-like domains (Ig1-2) or the first to third Ig-like domains (Ig1-3).

In an embodiment of the present invention, the PTPsigma-derived protein may comprise residues at positions 30-231 of PTPsigma protein, as an amino acid sequence derived from Ig1-2 of the extracellular domain of PTPsigma. In this case, the amino acid sequence number is based on isoform 3 (Q13332-7) of the PTPsigma protein.

Specifically, the PTPsigma-derived protein may comprise the following amino acid sequence of SEQ ID NO: 1, as residues at positions 30-231 of the PTPsigma protein:

[SEQ ID NO: 1]

EEPPRFIKEPKDQIGVSGGVASFVCQATGDPKPRVTWNKKGKKVNSQRFETI
EFDESAGAVLRIQPLRTPRDENVYECVAQNSVGEITVHAKLTVLREDQLPSGFPNIDM
GPQLKVVERTRTATMLCAASGNPDPEITWFKDFLPVDPSASNGRIKQLRSGALQIESS
EETDQGKYECVATNSAGVRYSSPANLYVRVRRVA

In an embodiment of the present invention, the PTPsigma-derived protein may comprise residues at positions 30-321 of the PTPsigma protein, as an amino acid sequence derived from Ig1-3 of the extracellular domain of PTPsigma.

Residues at positions 30-321 of the PTPsigma protein are residues in which the amino acid sequence of SEQ ID NO: 1 is followed by residues at positions 232-321 of the PTPsigma protein, and may be represented as follows:

[30-321 of the PTPsigma protein]

EEPPRFIKEPKDQIGVSGGVASFVCQATGDPKPRVTWNKKGKKVNSQRFETI
EFDESAGAVLRIQPLRTPRDENVYECVAQNSVGEITVHAKLTVLREDQLPSGFPNIDM
GPQLKVVERTRTATMLCAASGNPDPEITWFKDFLPVDPSASNGRIKQLRSGALQIESS
EETDQGKYECVATNSAGVRYSSPANLYVRVRRVAPRFSILPMSHEIMPGGNVNITCV
AVGSPMPYVKWMQGAEDLTPEDDMPVGRNVLELTDVKDSANYTCVAMSSLGVIEA
VAQITVKSLPKA

In the PTPsigma-Fc fusion protein of the present invention, a PTPsigma mutant for improving solubility in aqueous solution of the fusion protein may be formed in the PTPsigma-derived protein.

In the present invention, the term "mutation" is meant to include substitutions, insertions and/or deletions of amino acid residues, and preferably, the mutants of the present invention include substitutions of amino acid residues. Substitution of an amino acid residue may be represented by the sequence of the residue in the parent amino acid sequence, the number of the amino acid residue, and the amino acid residue substituted.

A fusion protein comprising an Ig-like domain as in the present invention does not have high solubility in aqueous solution, and thus, it is is difficult to process at a high dose in animals or humans. In order to solve this problem, solubility was improved in the present invention by selecting amino acids that do not change the structure among hydrophobic amino acids exposed on the surface of the Ig-like domain of PTPsigma and substituting them with hydrophilic amino acids.

Specifically, the PTPsigma mutant for improving the solubility of the fusion protein may be one in which one or more residues of isoleucine (I), valine (V), leucine (L) and tyrosine (Y) in the PTPsigma-derived protein are substituted with alanine (A), serine (S), threonine (T), asparagine (N), glutamine (Q), aspartic acid (D), glutamic acid (E), lysine (K), arginine (R), or histidine (H).

Among them, one or more residues of 136, V104, V108, L125, L143, L145, L155, L187, Y224 and V227 at positions 30-231 of PTPsigma are preferably substituted with A, S, T, N, Q, D, E, K, R, or H in terms of not affecting the structure of PTPsigma, and one or more residues of L143, L155, L187, Y224 and V227 are more preferably substituted with A or N in terms of improving solubility.

That is, residue 30 of the PTPsigma sequence corresponds to residue 1 of SEQ ID NO: 1 based on the amino acid sequence of SEQ ID NO: 1 of the present invention, and thus, one or more residues of I7, V75, V79, L96, L114, L116, L126, L158, Y195 and V198 in SEQ ID NO: 1 may be substituted with A, S, T, N, Q, D, E, K, R or H, and more preferably, one or more residues of L114, L126, L158, Y195 and V198 in SEQ ID NO: 1 may be substituted with A or N.

In the experimental examples of the present invention, it was confirmed that the solubility in aqueous solution of the fusion protein increased up to 6-fold by the mutation, and the effect of promoting the regeneration of blood stem cells was excellent. Accordingly, the PTPsigma-Fc fusion protein into which the mutation is introduced is expected to be used as a composition for promoting the regeneration of blood stem cells, and to be treated at a high-dose in the treatment of humans and animals.

In the present invention, the PTPsigma-derived protein is linked to an immunoglobulin-derived Fc domain to form a PTPsigma-Fc fusion protein.

In the present invention, the immunoglobulin-derived Fc domain refers to a C-terminal region including CH2 and CH3 domains (or CH2, CH3 and CH4 domains) of the immunoglobulin heavy chain constant region, and is used as a meaning encompassing the wild-type Fc domain and its mutant. The parent immunoglobulin of the Fc domain may be IgG1, IgG2, IgG3, or IgG4, and preferably may be IgG1.

In the present invention, each chain of the Fc domain may include a region extending from residue 221 of a human IgG1 heavy chain to the C-terminus, or a region further including a hinge in the region. The number of amino acid residues in the Fc region is according to Kabat EU numbering, which defines the number of residues in human immunoglobulin heavy chains.

In the present invention, each chain of the Fc domain may include 221-447 residues of the IgG1 heavy chain. 221-447 residues of the IgG1 heavy chain may be represented by the amino acid sequence of following SEQ ID NO: 2:

[SEQ ID NO: 2]

DKTHTCPPCPAPELLGGPSVFLFPPKPKDTLMISRTPEVTCVVVDVSHEDPEV
KFNWYVDGVEVHNAKTKPREEQYNSTYRVVSVLTVLHQDWLNGKEYKCKVSNKA
LPAPIEKTISKAKGQPREPQVYTLPPSRDELTKNQVSLTCLVKGFYPSDIAVEWESNG
QPENNYKTTPPVLDSDGSFFLYSKLTVDKSRWQQGNVFSCSVMHEALHNHYTQKSL
SLSPGK

In the present invention, the PTPsigma-derived protein may be in a form linked to one or more of two chains constituting the Fc domain.

In this case, the PTPsigma-derived protein and the Fc domain may be linked by 0 to 20 amino acid residues. That is, the PTPsigma-derived protein and the Fc domain may be directly linked, or linked through a linker consisting of 1 to 20 amino acids. In this case, the PTPsigma-Fc-derived protein may be linked to the N-terminus or C-terminus of the Fc domain.

The PTPsigma-Fc fusion protein of the present invention may be a homodimer in which the PTPsigma-derived proteins are linked to both chains of the Fc domain, or a heterodimer in which the PTPsigma-derived protein is linked to only one chain.

In an embodiment of the present invention, when the PTPsigma-Fc fusion protein is a heterodimer, a recombinant mutant for forming a dimer may be formed in the Fc domain.

For example, the recombinant mutant for forming a dimer may be formed using a knobs-into-hole technology.

The knob-into-hole technology is a technology designed to form only heterodimers between heavy chains of antibody fragments. In this case, the knob is designed to have a side chain protruding from the opposite chain, and is inserted into a hole of the opposite domain. Due to this, heavy chains cannot be homodimerized by side chain collision, but can only be heterodimerized. In the present invention, a structure in which knobs or holes are formed in each chain of the Fc domain may be referred to as an Fc-knob or an Fc-hole, respectively.

The Fc-knob may be formed by substituting one or more amino acids in the chain constituting the Fc domain with a large amino acid selected from the group consisting of tryptophan (W), arginine (R), phenylalanine (F) and tyrosine (Y). For example, the Fc-knob may have T366W mutation formed in positions 221-447 of the IgG1-Fc heavy chain.

The Fc-hole may be formed by substituting one or more amino acids in the chain constituting the Fc domain with a small amino acid selected from the group consisting of alanine (A), serine (S), threonine (T) and valine (V). For example, the Fc-hole may have T366S, L368A and Y407V mutations formed in positions 221-447 of the IgG1-Fc heavy chain.

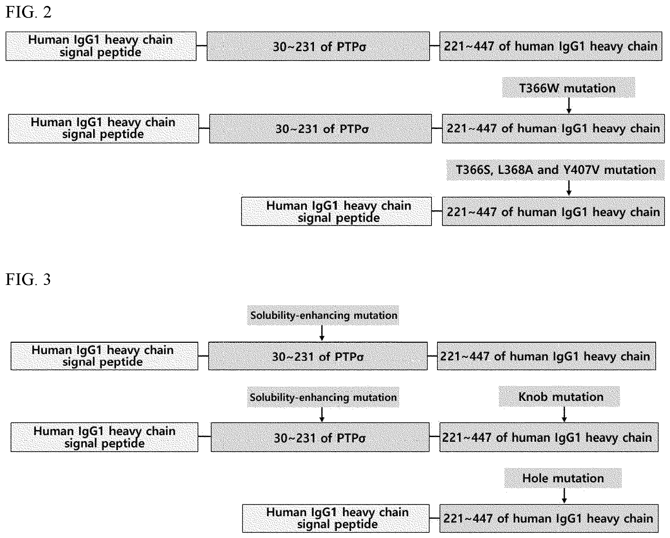

FIG. 2 shows an amino acid sequence map of polypeptide chains constituting the PTPsigma-Fc fusion protein according to an embodiment of the present invention.

As shown in FIG. 2, the PTPsigma-Fc fusion protein of the present invention may comprise a polypeptide consisting of a heavy chain signal peptide, residues at positions 30-231 of PTPsigma, and residues at positions 221-447 of the IgG1 heavy chain, and in this case, a homodimeric fusion protein may be formed. Alternatively, the PTPsigma-Fc fusion protein of the present invention may comprise a polypeptide consisting of a heavy chain signal peptide, residues at positions 30-231 of PTPsigma, and a knob mutant of residues at positions 221-447 of the IgG1 heavy chain, and a heavy chain signal peptide and a hole mutant of residues at positions 221-447 of the IgG1 heavy chain, and in this case, a heterodimeric fusion protein may be formed.

FIG. 3 shows an amino acid sequence map of a solubility-enhancing mutant of the PTPsigma-Fc fusion protein according to an embodiment of the present invention, i.e., a PTPsigma mutant-Fc fusion protein.

As shown in FIG. 3, the PTPsigma mutant-Fc fusion protein of the present invention may comprise a polypeptide consisting of a heavy chain signal peptide, a solubility-enhancing mutant of residues at positions 30-231 of the PTPsigma, and residues at positions 221-447 of the IgG1 heavy chain, and in this case, a homodimeric fusion protein may be formed. Alternatively, the PTPsigma mutant-Fc fusion protein of the present invention may comprise a polypeptide consisting of a heavy chain signal peptide, a solubility-enhancing mutant of residues at positions 30-231 of the PTPsigma, and a knob mutant of residues at positions 221-447 of the IgG1 heavy chain, and a heavy chain signal peptide and a hole mutant of residues at positions 221-447 of the IgG1 heavy chain, and in this case, a heterodimeric fusion protein may be formed.

The use of the PTPsigma-Fc fusion protein formed according to the present invention may block the reaction between the extracellular domain of PTPsigma and its ligand, and thus, it may be used as an inhibitor for suppressing signaling by PTPsigma.

Accordingly, the present invention also provides a composition comprising the PTPsigma-Fc fusion protein.

In the present invention, it was confirmed that the PTPsigma-Fc fusion protein may block the receptor-ligand interaction between the extracellular domain of PTPsigma and its ligand, thereby suppressing blood stem cell growth-inhibiting signaling by PTPsigma. Accordingly, the PTPsigma-Fc fusion protein of the present invention may promote the growth of blood stem cells, and specifically, may be used to promote the regeneration of blood stem cells whose growth was inhibited by irradiation and use of chemical anticancer drugs.

Therefore, the PTPsigma-Fc fusion protein of the present invention is expected to be effective in helping recovery of cancer patients in need of radiotherapy and chemical cancer drugs. When the blood stem cells of patients suffering from cancer such as, for example, pancreatic cancer, liver cancer, breast cancer, brain cancer, prostate cancer, colorectal cancer, lung cancer, kidney cancer, neuroblastoma, ovarian cancer, endometrial cancer, germinoma, eye cancer, multiple myeloma and stomach cancer are damaged by radiotherapy and/or anticancer drugs, it may be used as a therapeutic agent for rapid recovery of cancer patients by helping to regenerate the damaged blood stem cells.

In addition, the extracellular domain of PTPsigma is known to be involved in nerve cell regeneration-interfering signaling, immune response-activating signaling, and the like through interaction with chondroitin sulfate proteoglycan (CSPG), and thus, the PTPsigma-Fc fusion protein of the present invention may be used in a pharmaceutical composition for preventing or treating a PTPsigma-mediated disease.

Specifically, the PTPsigma-Fc fusion protein of the present invention may be used to prevent or treat PTPsigma-mediated nerve injury diseases such as traumatic brain injury, Alzheimer's dementia and Parkinson's disease, and may be used to prevent or treat a PTPsigma-mediated synaptic plasticity-related disease selected from the group consisting of mania, depression, manic depression, and amnestic disorder. In addition, the PTPsigma-Fc fusion protein of the present invention may be used for preventing or treating PTPsigma-mediated autoimmune diseases such as rheumatoid arthritis or multiple sclerosis.

The PTPsigma-Fc fusion protein of the present invention is used for protein-protein interaction, and thus, it may overcome problems of inhibitory strength of enzyme function, selectivity, permeability of a cell membrane, and the like, that are inherent in small molecule inhibitors targeting the active site of PTP. In addition, in the present invention, solubility in aqueous solution may be improved by introducing mutations into the PTPsigma-Fc fusion protein, ant thus, it is expected that it may be treated at a high dose in human and animal treatment.

The composition of the present invention may comprise a pharmaceutically acceptable carrier in addition to the PTPsigma-Fc fusion protein.

The pharmaceutically acceptable carriers are those commonly used in formulations and include, but are not limited to, lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methylcellulose, methylhydroxybenzoate, propylhydroxybenzoate, talc, magnesium stearate, and mineral oil. The pharmaceutical composition of the present invention may further comprise a lubricant, a wetting agent, a sweetener, a flavoring agent, an emulsifier, a suspending agent, a preservative, and the like, in addition to the ingredients as described above.

The composition comprising the PTPsigma-Fc fusion protein of the present invention may be administered orally or parenterally, and for parenteral administration, it may be administered by intravenous injection, subcutaneous injection, intramuscular injection, intraperitoneal injection, endothelial administration, topical administration, intranasal administration, intrapulmonary administration, intrarectal administration, and the like.

The composition comprising the PTPsigma-Fc fusion protein of the present invention may be formulated in the form of a sterile injection solution, a lyophilized formulation, a prefilled syringe solution, an oral formulation, an external preparation or a suppository, according to conventional methods. When administered orally, the oral composition may be formulated to coat the active agent or protect it from digestion in the stomach because the protein or peptide is digested.

The appropriate dosage of the PTPsigma-Fc fusion protein of the present invention may be variously prescribed depending on factors such as formulation method, the mode of administration, the age, body weight, sex and pathological condition of the patient, diet, the time of administration, the route of administration, the rate of excretion, and response sensitivity. For example, the daily dose of the PTPsigma-Fc fusion protein may be, for example, 0.001 to 100 mg/kg.

The present invention also relates to a method of preventing or treating a PTPsigma-mediated disease, wherein the method may comprise administering a pharmaceutical composition comprising the PTPsigma-Fc fusion protein.

The present invention also relates to a method of promoting the regeneration of blood stem cells, wherein the method may comprise administering a pharmaceutical composition comprising the PTPsigma-Fc fusion protein. The method may be used for promoting the regeneration of blood stem cells damaged by irradiation or administration of anticancer drugs in cancer patients.

In the present invention, the subject to be administered may be a subject, specifically, a subject in need of administration of the PTPsigma-Fc fusion protein, and the subject may be an animal, usually a mammal.

### EXAMPLES

Hereinafter, the present invention will be described in more detail through examples. However, these Examples show some experimental methods and compositions only for illustrating the present invention, and the scope of the present invention is not limited to these Examples.

### Preparative Example 1: Preparation of PTPsigma-Fc fusion proteins

### 1-1. Construction of expression vectors

An expression vector was constructed based on pcDNA 3.1/Myc His-A (Invitrogen) to have a polyhistidine tag at the C-terminus of the protein.

A polynucleotide encoding the amino acid sequence of the target protein was amplified by PCR technique, and then cloned through restriction enzymes and ligases. Through this, an Fc expression vector expressing residues at positions 221-447 of the human immunoglobulin G1 (IgG1) heavy chain including the signal peptide of the human immunoglobulin G1 heavy chain was constructed. In this case, the amino acid sequence number of the Fc protein is based on "EU numbering."

A restriction enzyme recognition sequence was inserted into the vector through mutagenesis to insert nucleotides encoding residues at positions 30-231 of Uniprot Q133332-7 PTPsigma. Through this, a PTPsigma-Fc expression vector was constructed.

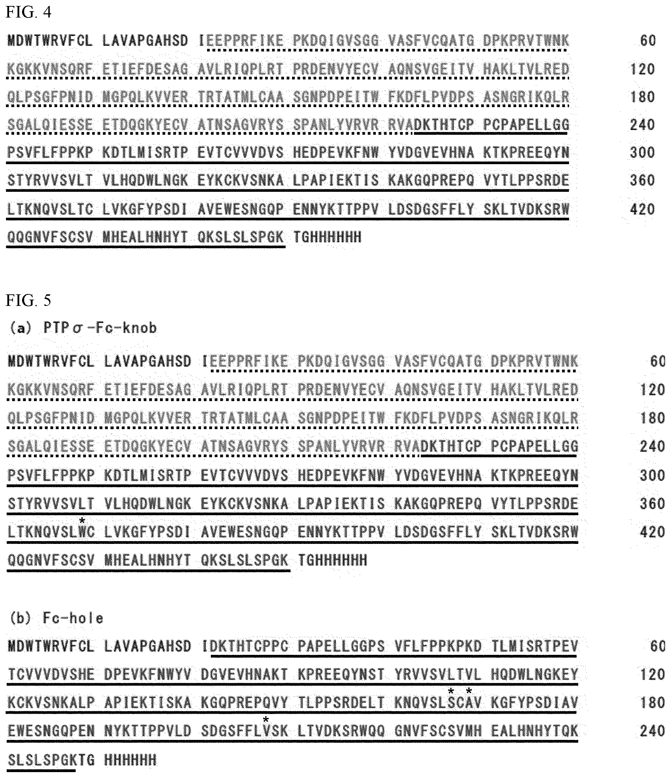

The amino acid sequence encoded by the PTPsigma-Fc expression vector is as shown in FIG. 4. In FIG. 4, the part underlined with a dotted line corresponds to the PTPsigma-derived protein (SEQ ID NO: 1), and the part underlined with a straight line corresponds to IgG1 221-447 (SEQ ID NO: 2) in the Fc domain.

Based on the vector constructed above, mutants for forming knobs and holes capable of producing heterodimeric proteins were introduced.

A PTPsigma-Fc-knob expression vector was constructed by changing the nucleotide sequence so that the knob due to T366W mutation was formed in the PTPsigma-Fc expression vector.

An Fc-hole expression vector encoding the amino acid sequence as shown below was constructed by changing the nucleotide sequence so that holes due to T366S, L368A and Y407V mutations were formed at positions 221-447 of the IgG1 heavy chain in the Fc expression vector.

FIG. 5 shows amino acid sequences encoded by (a) the PTPsigma-Fc-knob expression vector and (b) the Fc-hole expression vector. In FIG. 5, the part underlined with a dotted line corresponds to the PTPsigma-derived protein (SEQ ID NO: 1), and the parts underlined with straight lines correspond to the knob or hole mutants of IgG1 221-447 (SEQ ID NO: 2) in the Fc domain. Mutated residues are marked with an asterisk.

### 1-2. Protein expression

Expression vectors were transfected into ExpiCHO^{™} cells to transiently express each protein.

The transfection was performed under conditions of an initial cell density of 6×10⁶ cells/mL and an expression vector concentration of 0.8 µg DNA/mL, and ExpiCHO^{™} Expression Medium, OptiPRO^{™} SFM and ExpiFectamine^{™} transfection kit (Gibco) were used in this process according to the manufacturer's instructions.

The transfected cells were cultured in a carbon dioxide incubator for 8 days, and then separated into a cell culture broth containing each protein and cells by centrifugation.

### 1-3. Purification

In order to separate target proteins from the culture broth containing each recombinant protein, affinity chromatography was performed using a Ni-NTA (Qiagen) column.

Specifically, the Ni-NTA column was filled with a buffer solution containing 50 mM Tris-HCl, pH 7.5 and 200 mM NaCl, and then, the culture broth obtained through centrifugation was filtered through 0.45 µm filter paper and introduced. The reaction with the culture broth was completed, and then, the column was washed sequentially with a buffer solution containing 50 mM Tris-HCl, pH 7.5 and 500 mM NaCl, and a buffer solution containing 50 mM Tris-HCl, pH 7.5, 200 mM NaCl and 30 mM imidazole. Finally, proteins bound to the column were eluted using a buffer solution containing 50 mM Tris-HCl, pH 7.5, 200 mM NaCl, and 500 mM imidazole. After elution, the composition of the buffer solution containing the protein was replaced with phosphate buffered saline (PBS) using a Hitrap^{™} Desalting (GE healthcare) column.

### 1-4. Combination of heterodimeric proteins

After quantification of purified PTPsigma-Fc-knob protein and Fc-hole protein, they were mixed in the same molecular ratio. Reagents were added to the mixed protein solution so that the final concentrations were 0.1 M arginine and 5 mM L-glutathione (reduced), and reacted at 37°C for 2 hours to form heterodimeric proteins. SDS-PAGE experiments were performed on the protein after the reaction was completed.

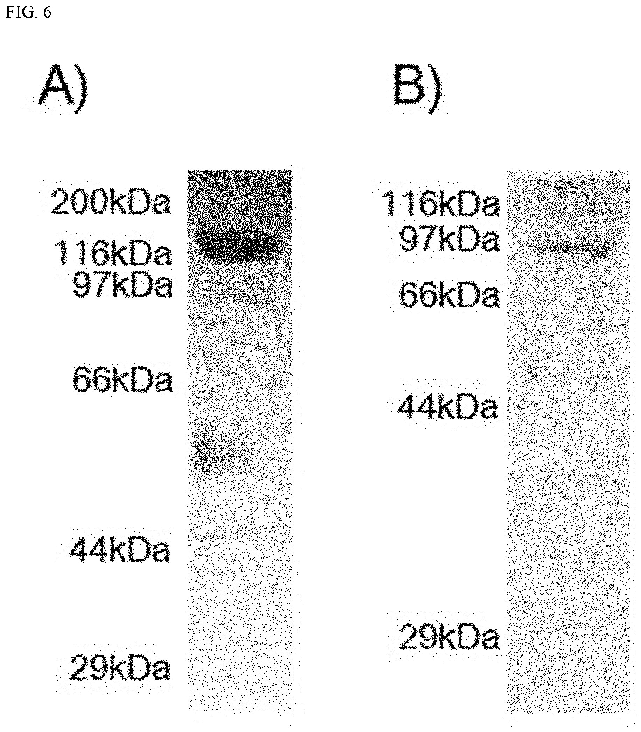

FIG. 6 shows the SDS-PAGE results of homodimeric and heterodimeric proteins formed in Preparative Example 1. FIG. 6A shows the result for the PTPsigma-Fc homodimer, and FIG. 6B shows the result for the PTPsigma-Fc-knob:Fc-hole heterodimer. It was confirmed that the yield of the PTPsigma-Fc homodimer was about 6 mg/L, and the PTPsigma-Fc-knob:Fc-hole heterodimer was formed with about 85% purity.

### Experimental Example 1: Measurement of the effect of PTPsigma-Fc fusion proteins on the regeneration of blood stem cells through colony-forming cell assay

As cells for the colony-forming cell assay of hematopoietic stem cells, commercially available human CD34+ hematopoietic stem cells (STEMCELL Technologies) were used.

The human CD34+ hematopoietic stem cells were cultured under conditions of 37°C and 5% carbon dioxide concentration immediately after thawing, using StemSpan^{™} SFEM and StemSpan^{™} CD34+ Expansion Supplement (10×) (STEMCELL Technologies) according to the manufacturer's instructions.

Cultured human CD34+ hematopoietic stem cells were seeded in a 96-well microplate (SPL Life Science) at a cell concentration of 2×10³ cells/well. In this case, MethoCult^{™} H4434 Classic (STEMCELL Technologies) culture broth was used for the colony-forming cell assay. Immediately after seeding, the fusion protein of Preparative Example 1 and PBS were applied to each well. 24 hours after seeding, the hematopoietic stem cells were irradiated with 300 cGy of radiation using a Cs-137 irradiator and damaged.

After irradiation, colony-forming cell assay were performed using the CytoSelect^{™} 96-Well Hematopoietic Colony Forming Cell Assay (Cell Biolabs) kit product. Specifically, the reagents of the colony-forming cell assay kit were mixed with hematopoietic stem cells cultured under conditions of 37°C and 5% carbon dioxide concentration for 9 days after irradiation, and then, fluorescence was measured at a wavelength of 355 nm/460 nm using a Victor X2 (Perkin-Elmer) plate meter.

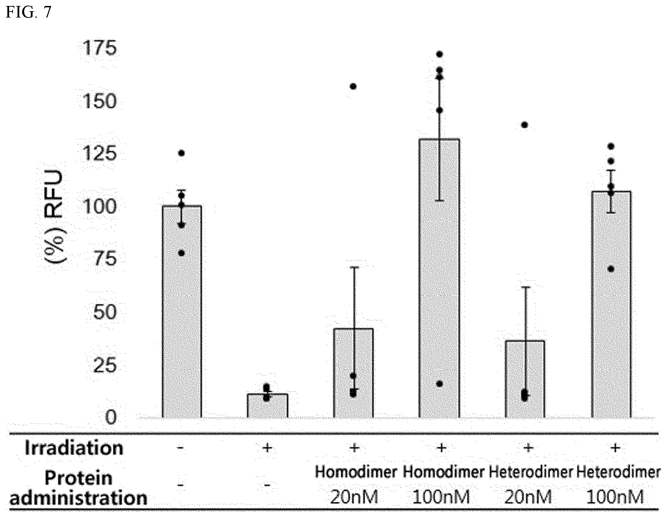

FIG. 7 shows the results for the colony-forming cell assay. It can be seen that when hematopoietic stem cells are irradiated with radiation, colony-forming cells are greatly reduced. However, it was confirmed that when hematopoietic stem cells were treated with the homodimer of PTPsigma-Fc according to the present invention, colony-forming cells were restored almost as in the control group. Similar results were obtained even when they were treated with the heterodimer consisting of PTPsigma-Fc-knob:Fc-hole.

Accordingly, it was confirmed that the PTPsimga-Fc fusion protein of the present invention may be used to effectively restore damaged hematopoietic stem cells after radiotherapy in cancer patients.

### Preparative Example 2: Preparation of mutants of PTPsigma-Fc fusion proteins

In order to improve the solubility of the fusion protein, a mutant was designed in the PTPsigma-derived protein.

Specifically, the mutation was designed based on the tertiary structure of PTPsigma, wherein the mutation was designed so that the CSPG binding ability of PTPsigma was not impaired, and thus, the inhibitory effect of PTPsigma was not affected.

Residues with the Ig1-2 tertiary structure of the extracellular domain of PTPsigma were analyzed one by one with the PyMOL program, and residues with a large solvent accessible surface area and hydrophobicity were selected. Among them, residues that could affect the structure when a mutation was introduced because they interact with neighboring residues in structure were excluded.

Then, in order not to affect CSPG binding, the complex structure of PTP-lar homologous to PTPsigma and sucrose octasulfate, which is a CSPG analogue, was used as PTP-sulfate interaction information. First, the PTPsigma Ig1-2 structure was overlapped with the Ig1-2 structure of PTP-lar, and then, the residues of PTPsigma Ig1-2 at the site in which sucrose octasulfate interacts were excluded from the residues to be mutated.

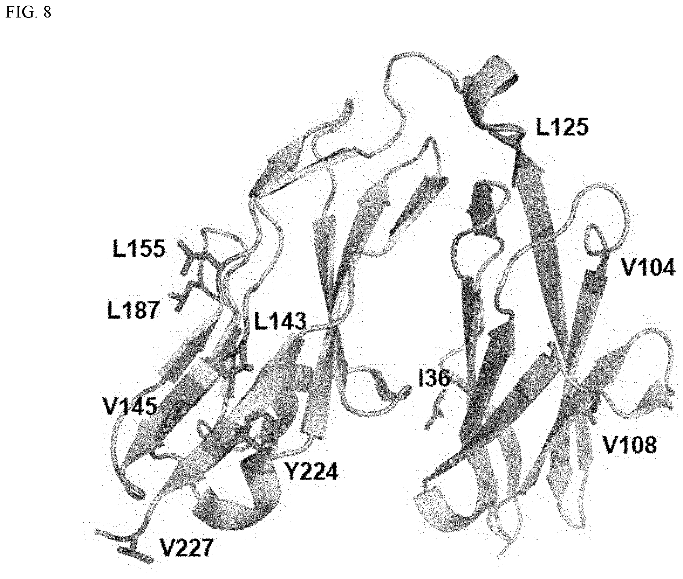

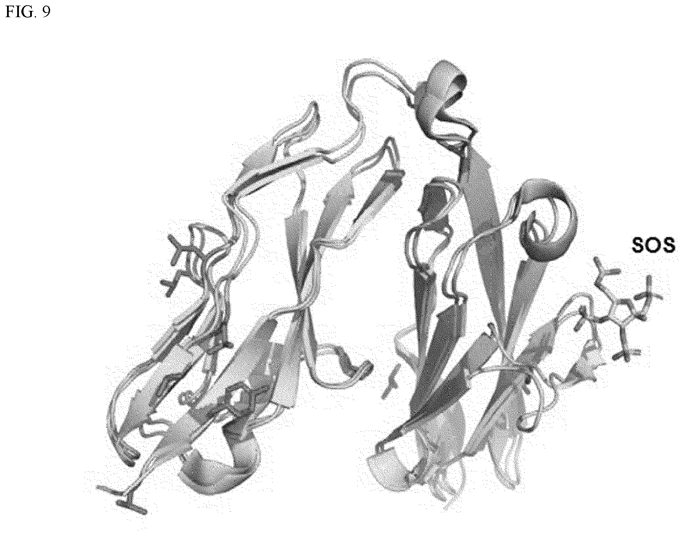

As a result, it was confirmed that the positions of the amino acids to be mutated were 136, V104, V108, L125, L143, L145, L155, L187, Y224 and V227 in the PTPsigma sequence, and FIG. 8 shows the positions of the residues in the tertiary structure of PTPsigma Ig1-2. In addition, FIG. 9 shows that the complex structure of Ig1-2 of PTP-lar and sucrose octasulfate and the tertiary structure of PTPsigma Ig1-2 were overlapped.

Solubility was improved by changing these amino acids to hydrophilic amino acids, and mutants consisting of combinations of one or more of these amino acids among them were used. Mutation to hydrophilic amino acids was performed by selecting one of A, S, T, N, Q, D, E, K, R and H amino acids, and the mutation positions and residues are shown in Table 1 below. In Table 1, the number of amino acid residues is based on the amino acid sequence number of PTPsigma.

**[Table 1]**

| Mutated position | Mutated residue |
|---|---|
| I36 | A, S, T, N, Q, D, E, K, R, H |
| V104 | A, S, T, N, Q, D, E, K, R, H |
| V108 | A, S, T, N, Q, D, E, K, R, H |
| L125 | A, S, T, N, Q, D, E, K, R, H |
| L143 | A, S, T, N, Q, D, E, K, R, H |
| V145 | A, S, T, N, Q, D, E, K, R, H |
| L155 | A, S, T, N, Q, D, E, K, R, H |
| L187 | A, S, T, N, Q, D, E, K, R, H |
| Y224 | A, S, T, N, Q, D, E, K, R, H |
| V227 | A, S, T, N, Q, D, E, K, R, H |

Based on the mutated positions and residues, representative sequences of amino acid mutations of PTPsigma Ig1-2 including mutations with improved solubility in aqueous solution were designed as shown in Table 2 below.

**[Table 2]**

| Mutant No. | Mutated residue |
|---|---|
| 1 | L143N, V145T, Y224N |
| 2 | L143A, V145A, Y224A |
| 3 | L143N, V145T, Y224N, V104T, V108T, L155N, L187N |
| 4 | V104T, V108T, L155N, L187N |
| 5 | L143N, V145T, Y224N, V104T |
| 6 | L143N, V145T, Y224N, V108T |
| 7 | L143N, V145T, Y224N, L155N |
| 8 | L143N, V145T, Y224N, L187N |
| 9 | L143A, V145A, Y224A, V104A, V108A, L155A, L187A |
| 10 | V104A, V108A, L155A, L187A |
| 11 | L143A, V145A, Y224A, V104A |
| 12 | L143A, V145A, Y224A, V108A |
| 13 | L143A, V145A, Y224A, L155A |
| 14 | L143A, V145A, Y224A, L187A |
| 15 | L155A |
| 16 | L155A, L187A, Y224A |
| 17 | L155N, L187N |
| 18 | L143A, L145A, L155A, L187A |

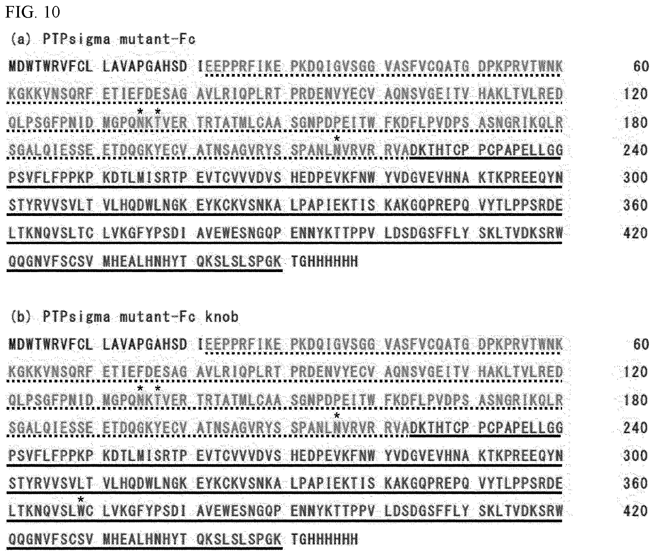

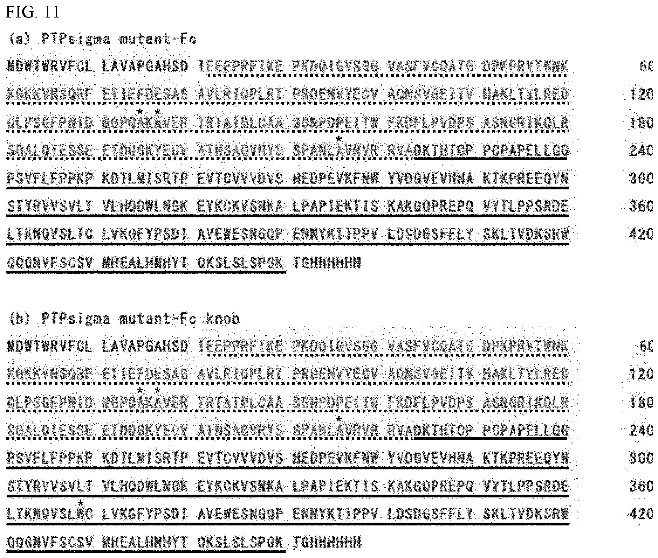

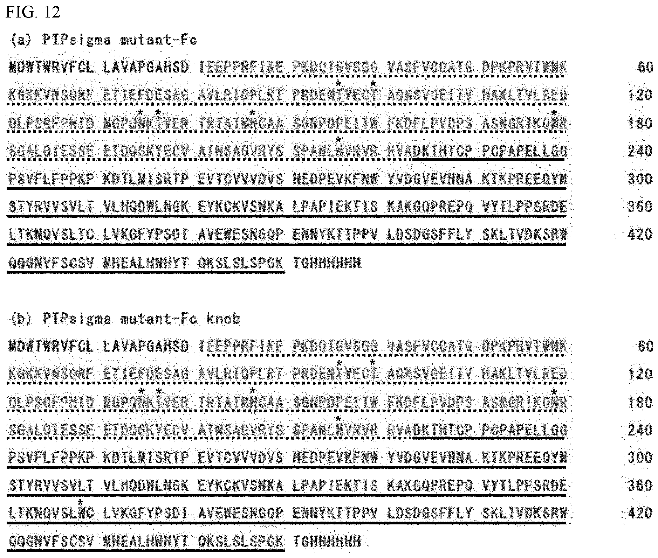

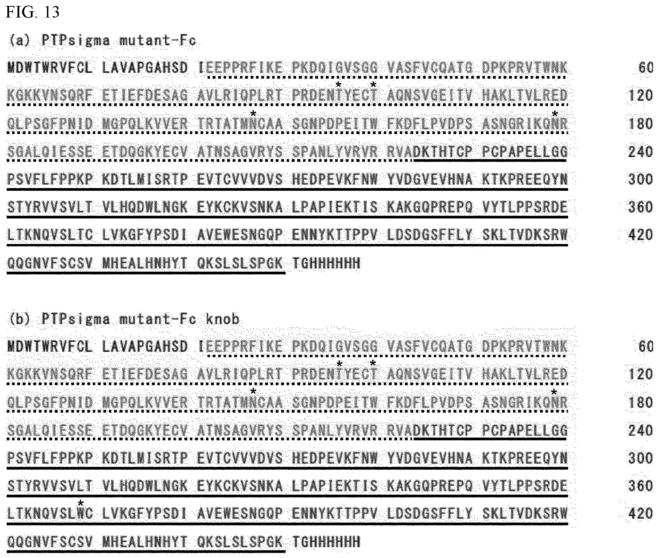

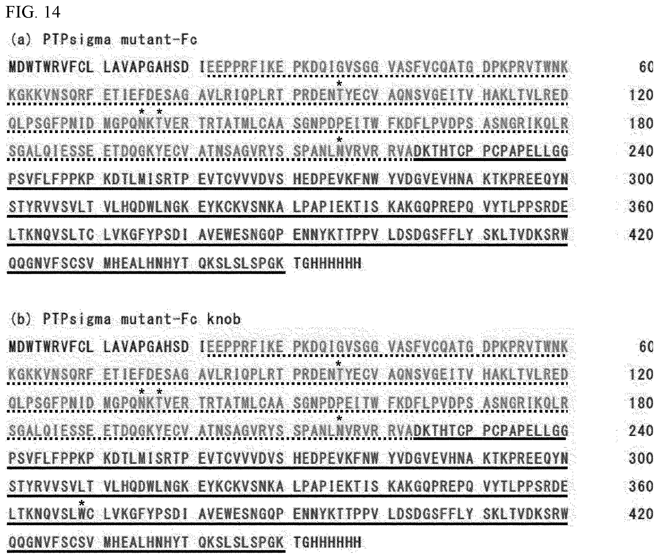

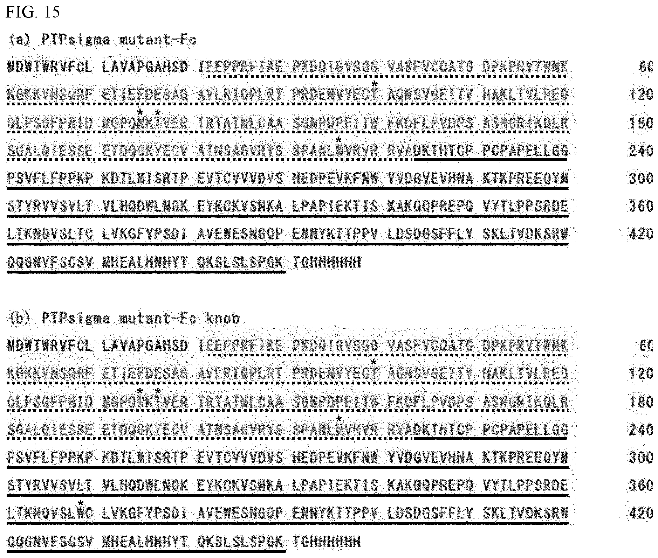

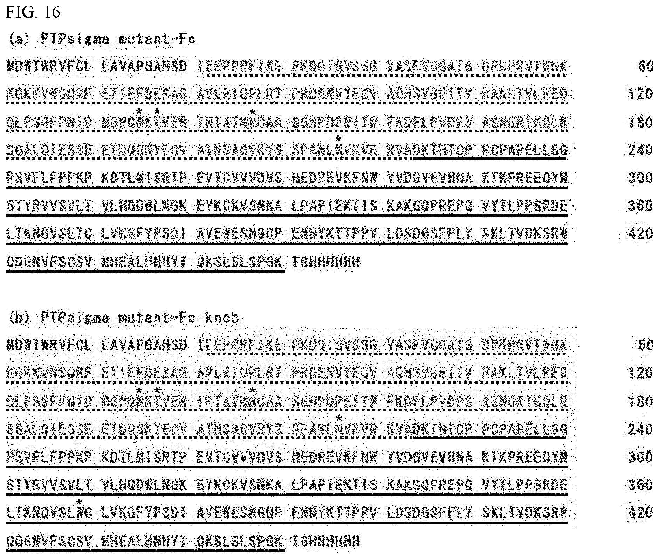

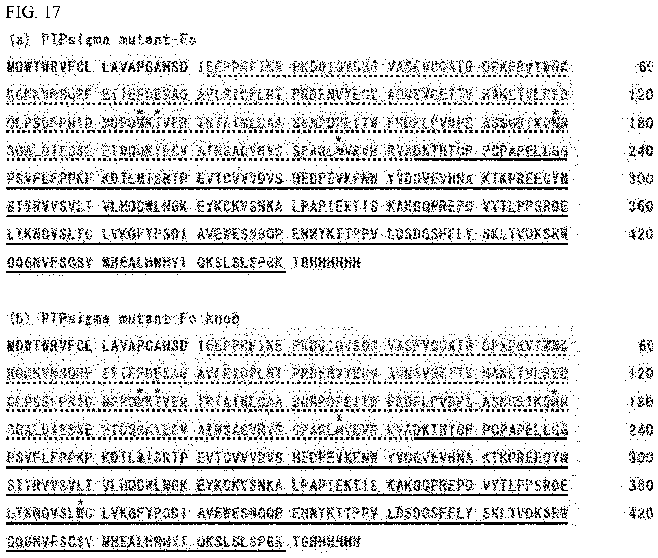

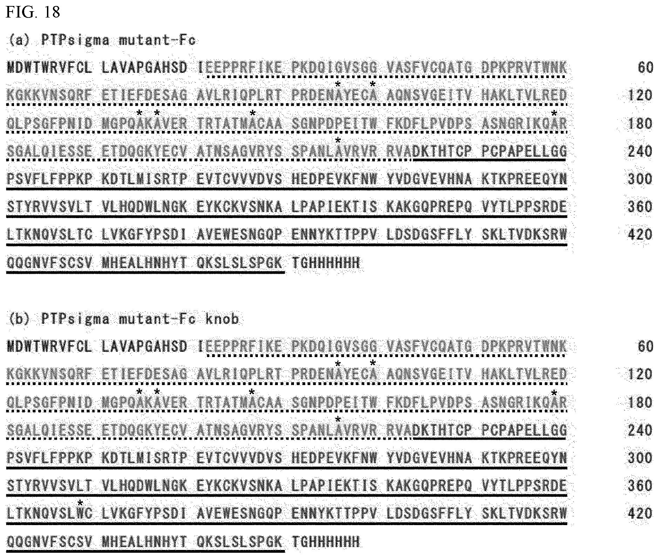

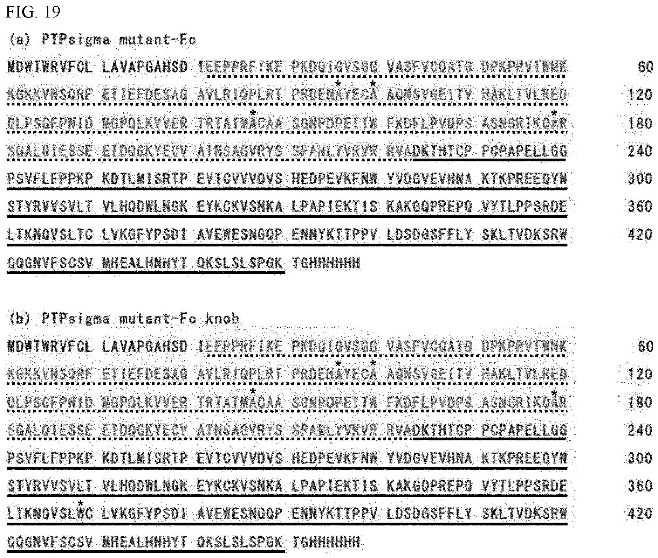

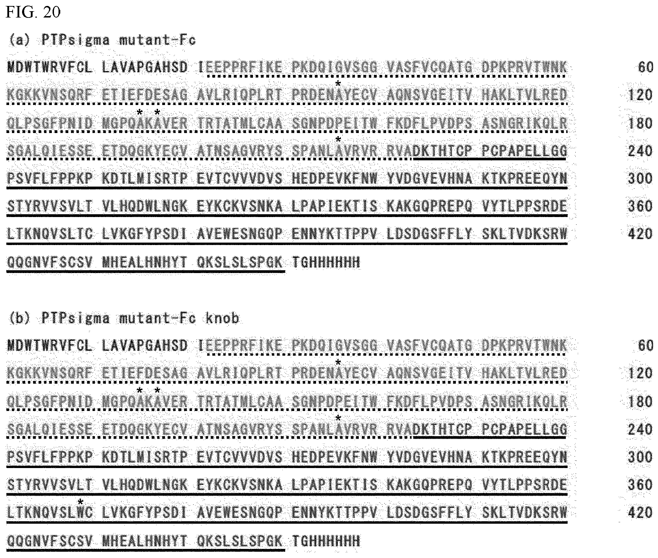

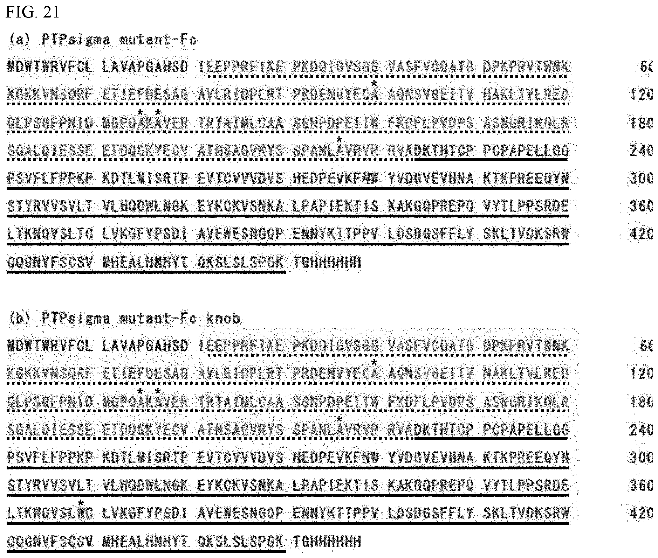

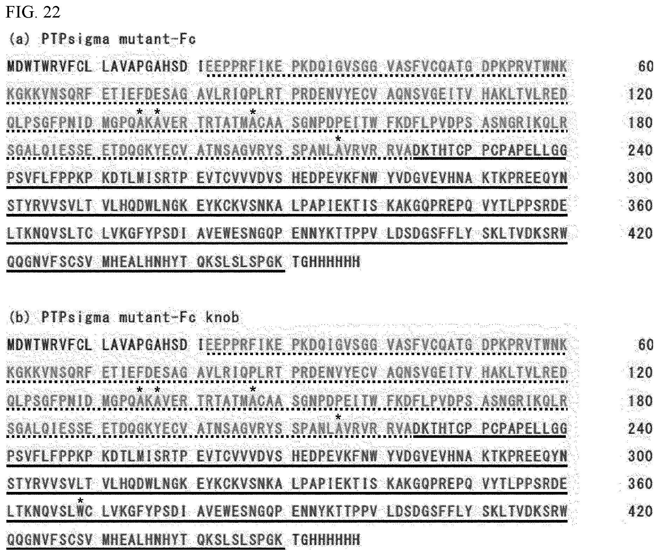

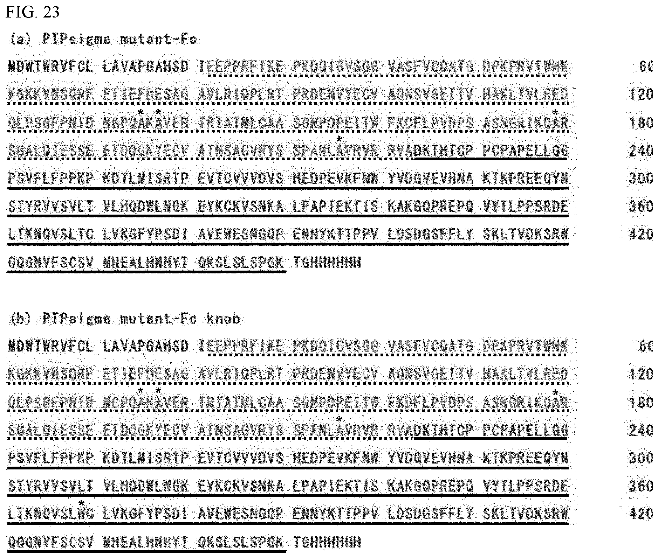

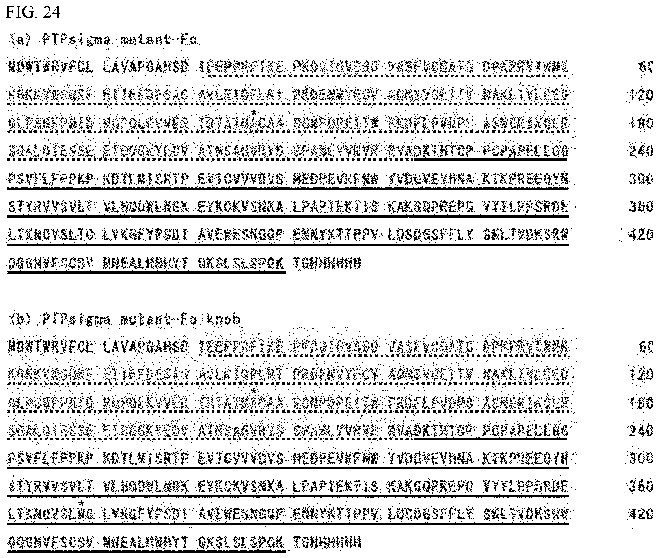

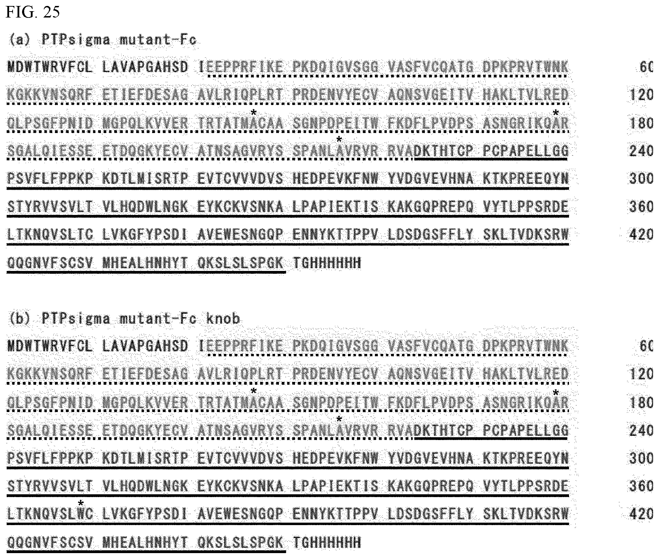

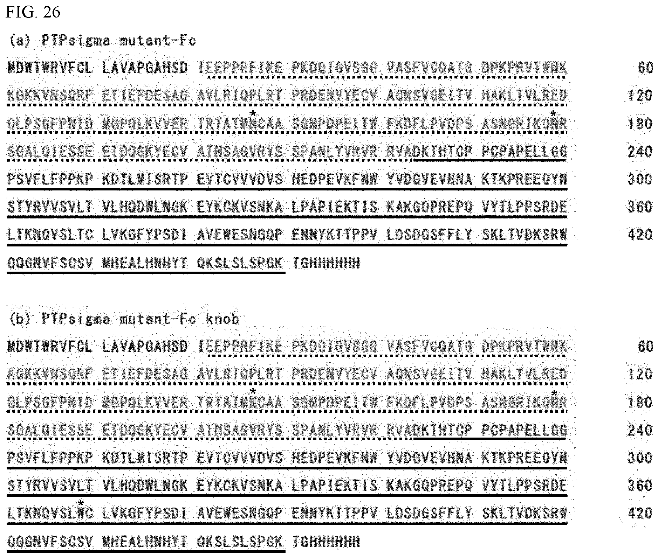

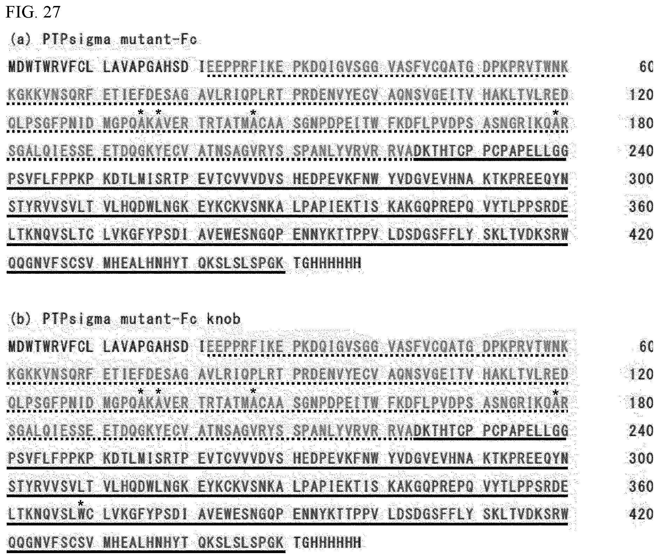

FIGS. 10 to 27 show amino acid sequences encoded by vectors for forming fusion proteins of each PTPsigma mutant and the Fc domain for representative mutant sequences of Table 2 above. In each sequence, (a) shows a sequence for forming a homodimeric fusion protein of PTPsigma mutant-Fc, and (b) shows a sequence of PTPsigma mutant-Fc knob for forming an asymmetric heterodimer.

In FIGS. 10 to 27, the parts underlined with a dotted line correspond to each mutant sequence of residues at positions 30-231 (SEQ ID NO: 1) of PTPsigma, which is the PTPsigma-derived protein, and the parts underlined with straight lines correspond to the IgG1 221-447 (SEQ ID NO: 2) of the Fc domain or its knob mutants. Residues mutated in the parent are marked with an asterisk.

### Experimental Example 2: Measurement of yield and solubility of PTPsigma-Fc fusion protein mutants

For the mutant fusion protein designed in the same way as in Preparative Example 2, the yield and solubility were measured and compared with the yield and solubility of the fusion protein (wild type) of Preparative Example 1.

In order to measure the yield of the PTPsigma-Fc fusion protein containing the mutant, 8ml of the elution was concentrated to close to 1ml immediately after purification, and then, centrifugation was performed to settle the insoluble contents, and the supernatant sample was taken. Then, the concentration of the supernatant sample was measured using Detergent Compatible assay (DC assay), and the amount of soluble protein was calculated based on the concentration and volume.

In addition, in order to measure the solubility in aqueous solution of the PTPsigma-Fc fusion protein containing the mutant, a protein volume of 8 ml was concentrated to 1 ml immediately after protein purification, and the concentration of the supernatant was measured by DC assay after centrifugation. After concentrating it to 400 µl, the same process was repeated to measure the concentration of the supernatant, and then, the amount of soluble protein was calculated by multiplying each concentration by the volume, and converted into a ratio (%).

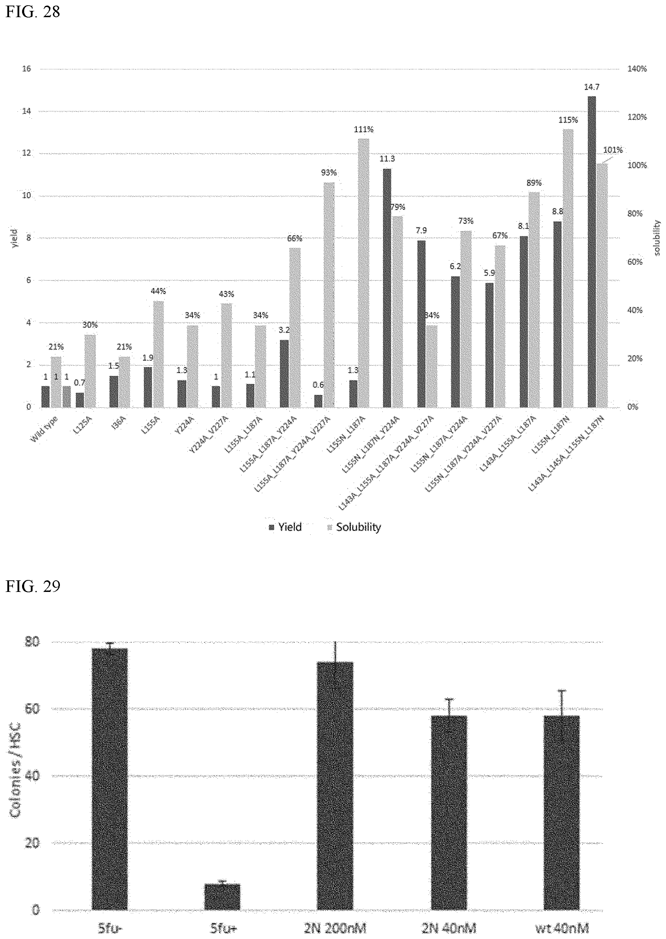

Yield and solubility results calculated by the above method are shown in Table 3 below and FIG. 28.

**[Table 3]**

| Mutation set | Yield | Solubility |
|---|---|---|
| Wild type | 1.0 | 21% |
| L125A | 0.7 | 30% |
| I36A | 1.5 | 21% |
| L155A | 1.9 | 44% |
| Y224A | 1.3 | 34% |
| Y224A, V227A | 1.0 | 43% |
| L155A, L187A | 1.1 | 34% |
| L155A, L187A, Y224A | 3.2 | 66% |
| L155A, L187A, Y224A, V227A | 0.6 | 93% |
| L155N, L187A | 1.3 | 111% |
| L155N, L187N, Y224A | 11.3 | 79% |
| L143A, L155A, L187A, Y224A, V227A | 7.9 | 34% |
| L155N, L187A, Y224A | 6.2 | 73% |
| L155N, L187A, Y224A, V227A | 5.9 | 67% |
| L143A, L155A, L187A | 8.1 | 89% |
| L155N, L187N | 8.8 | 115% |
| L143A, L145A, L155N, L187N | 14.7 | 101% |

From the yield and solubility measurement results, in the case of the mutants in which one or more residues of I36, V104, V108, L125, L143, L145, L155, L187, Y224 and V227 were substituted with A, S, T, N, Q, D, E, K, R or H, it was confirmed that the solubility in aqueous solution was improved compared to the wild type, and it was confirmed that the yield was also improved.

In particular, it was confirmed from the above experiments that the mutants in which one or more residues of L143, L155, L187, Y224 and V227 were substituted with A or N showed excellent results in terms of yield and solubility.

Accordingly, it was confirmed that the fusion protein of the present invention may be treated at a high dose in animals and humans by introducing specific mutations into PTPsigma-derived proteins to improve solubility.

### Experimental Example 3: Measurement of the effect of PTPsigma-Fc fusion protein mutants on the regeneration of blood stem cells

As cells for the colony-forming cell assay of hematopoietic stem cells, commercially available human CD34+ hematopoietic stem cells (STEMCELL Technologies) were used.

Immediately after thawing, the human CD34+ hematopoietic stem cells were cultured under conditions of 37°C and 5% carbon dioxide concentration, using StemSpan^{™} SFEM and StemSpan^{™} CD34+ Expansion Supplement (10×) (STEMCELL Technologies) according to the manufacturer's instructions.

Cultured human CD34+ hematopoietic stem cells were seeded in a 96-well microplate (SPL Life Science) at a cell concentration of 1.4×10³ cells/well. In this case, MethoCult^{™} H4434 Classic (STEMCELL Technologies) culture broth was used for the colony-forming cell assay. Immediately after seeding, the PTPsigma-Fc fusion protein and PBS were applied to each well. 24 hours after seeding, 5-fluorouracil (5FU) at a concentration of 5 µM was administered together with the fusion protein. After 14 days of 5FU treatment, the number of colonies was measured by manual counting method.

FIG. 29 shows the results of measuring the recovery effect of hematopoietic stem cell damage by anticancer drugs by the above method for wild type PTPsigma-Fc fusion proteins and PTPsigma-Fc fusion proteins containing L155N and L187N mutations. In FIG. 29, 5FU-represents a control group without addition of 5FU, 5FU+ represents a sample with 5FU added, 2N refers to a sample treated with PTPsigma-Fc fusion protein mutants (L155N and L187N) in a sample to which 5FU was added, and wt refers to a sample treated with PTPsigma-Fc fusion protein (wild type) in a sample to which 5FU was added.

Referring to FIG. 29, hematopoietic stem cells were severely damaged when treated with an anticancer drug (5FU), but it was confirmed that when the anticancer drug and PTPsigma mutant-Fc fusion protein were treated together, the damaged hematopoietic stem cells were recovered to a degree similar to those of the control group when not treated with the anticancer drug.

Accordingly, it was confirmed that the PTPsimga-Fc fusion protein of the present invention may be used to effectively regenerate damaged hematopoietic stem cells after treatment with anticancer drugs in cancer patients.

As a specific part of the present invention has been described in detail above, it will be apparent to those skilled in the art that these specific descriptions are only preferred embodiments, and the scope of the present invention is not limited thereby. Accordingly, it is intended that the substantial scope of the present invention be defined by the appended claims and equivalents thereof.

## Claims

1. A PTPsigma-Fc fusion protein comprising a PTPsigma (protein tyrosine phosphatase sigma)-derived protein linked to one or more of two chains constituting an immunoglobulin (Ig)-derived Fc domain.

2. The PTPsigma-Fc fusion protein according to claim 1, wherein the PTPsigma-derived protein is a protein derived from an extracellular domain (ectodomain) of PTPsigma.

3. The PTPsigma-Fc fusion protein according to claim 1, wherein the PTPsigma-derived protein is a protein derived from the first to second Ig domains (Ig1-2) or the first to third Ig domains (Ig1-3) of the extracellular domain of PTPsigma.

4. The PTPsigma-Fc fusion protein according to claim 1, wherein the PTPsigma-derived protein comprises amino acid residues 30-231 or amino acid residues 30-321 of PTPsigma.

5. The PTPsigma-Fc fusion protein according to claim 1, wherein one or more residues of isoleucine (I), valine (V), leucine (L) and tyrosine (Y) in the PTPsigma-derived protein are substituted with alanine (A), serine (S), threonine (T), asparagine (N), glutamine (Q), aspartic acid (D), glutamic acid (E), lysine (K), arginine (R), or histidine (H).

6. The PTPsigma-Fc fusion protein according to claim 1, wherein one or more residues of 136, V104, V108, L125, L143, L145, L155, L187, Y224 and V227 based on the amino acid sequence number of PTPsigma in the PTPsigma-derived protein are substituted with A, S, T, N, Q, D, E, K, R, or H.

7. The PTPsigma-Fc fusion protein according to claim 1, wherein the PTPsigma-Fc fusion protein is a homodimer or a heterodimer.

8. The PTPsigma-Fc fusion protein according to claim 1, wherein the chain constituting the Fc domain comprises amino acid residues 221-447 (based on Kabat EU numbering) of an IgG1 heavy chain.

9. The PTPsigma-Fc fusion protein according to claim 8, wherein:
one or more amino acids in one chain of the Fc domain are substituted with an amino acid selected from the group consisting of tryptophan (W), arginine (R), phenylalanine (F), and tyrosine (Y); and
one or more amino acids in the other chain are substituted with an amino acid selected from the group consisting of alanine (A), serine (S), threonine (T), and valine (V).

10. The PTPsigma-Fc fusion protein according to claim 8, wherein:
one chain of the Fc domain comprises T366W substitution (based on Kabat EU numbering); and
the other chain comprises T366S, L368A and Y407V substitutions (based on Kabat EU numbering).

11. A composition for promoting the growth of blood stem cells, comprising the PTPsigma-Fc fusion protein of any one of claims 1 to 10.

12. The composition for promoting the growth of blood stem cells according to claim 11, wherein the composition for promoting the growth of blood stem cells is used for regenerating blood stem cells damaged by irradiation or administration of anticancer drugs.

13. A pharmaceutical composition for preventing or treating a PTPsigma-mediated disease, comprising the PTPsigma-Fc fusion protein of any one of claims 1 to 10.

14. The pharmaceutical composition for preventing or treating a PTPsigma-mediated disease according to claim 13, wherein the PTPsigma-mediated disease is a nerve injury disease selected from the group consisting of traumatic brain injury, Alzheimer's dementia, and Parkinson's disease.

15. The pharmaceutical composition for preventing or treating a PTPsigma-mediated disease according to claim 13, wherein the PTPsigma-mediated disease is selected from the group consisting of multiple sclerosis and rheumatoid arthritis.

16. The pharmaceutical composition for preventing or treating a PTPsigma-mediated disease according to claim 13, wherein the PTPsigma-mediated disease is a synaptic plasticity-related disease selected from the group consisting of mania, depression, manic depression, and amnestic disorder.
